# EUROPÄISCHE PATENTSCHRIFT

(11) **EP 2 826 522 B1**
(45) Veröffentlichungstag und Bekanntmachung des Hinweises auf die Patenterteilung: **20.06.2018**
(21) Anmeldenummer: 14160543.6
(22) Anmeldetag: 18.03.2014
(51) Int. Cl.: A61N 1/362, A61N 1/39, A61N 1/365

(54) **HERZSCHRITTMACHER**
PACEMAKER
STIMULATEUR CARDIAQUE

(30) Priorität: 18.07.2013 DE 102013012059
(43) Veröffentlichungstag der Anmeldung: 21.01.2015
(73) Patentinhaber: Osypka AG, 79618 Rheinfelden-Herten (DE)
(72) Erfinder: BOLZ, Armin, 90154 Buckenhof (DE); KRAUSE, Jürgen, 91320 Ebermannstadt (DE); SEUFERT, Manuel, 91054 Erlangen (DE); TRÖGER, Tobias, 91052 Erlangen (DE); PLACKE, Volker, 91056 Erlangen (DE); BRAUN, Michael, 89231 Neu-Ulm (DE); OSYPKA, Peter, 79618 Rheinfelden-Herten (DE); GÖTTSCHE, Thorsten, 79618 Rheinfelden-Herten (DE); LOESCH, Thomas, 41179 Mönchengladbach (DE)
(74) Vertreter: Herrmann, Uwe

(56) Entgegenhaltungen:
- EP-A1- 0 824 938
- WO-A1-99/19021
- DE-U1-202010 011 244
- US-A- 5 324 310
- US-B1- 7 925 346
- INTISAR MIRZA ET AL: "Treatment of Atrial Fibrillation Biatrial Pacing for Paroxysmal Atrial Fibrillation A Randomized Prospective Study Into the Suppression of Paroxysmal Atrial Fibrillation Using Biatrial Pacing", JOURNAL OF THE AMERICAN COLLEGE OF CARDIOLOGY., vol. 40, no. 3, 2002, 7 August 2002 (2002-08-07), pages 457-463, XP055324048, US ISSN: 0735-1097

## Beschreibung

Die vorliegende Erfindung betrifft einen Herzschrittmacher zur Stimulation des Herzens.

Herzschrittmacher werden je nach Anwendungsfall in unterschiedlichen Ausführungsformen eingesetzt.

Externe Herzschrittmacher, d.h. Herzschrittmacher, die abgesehen von den Elektroden und ggf. einem Teil der zu diesen führenden Leitungen außerhalb des Körpers des Patienten angeordnet sind, werden beispielsweise zur temporären kardialen Stimulation und intrakardialen EKG-Überwachung nach herzchirurgischen Eingriffen eingesetzt. Sowohl die Stimulation als auch die Überwachung erfolgen üblicherweise mit Hilfe von Elektroden, die auf den Herzmuskel aufgebracht und vorzugsweise aufgenäht sind und die über Drähte mit dem externen Herzschrittmacher verbunden sind.

Darüber hinaus sind implantierte bzw. implantierbare Herzschrittmacher bekannt, die vollständig in dem Körper eingesetzt sind.

Darüber hinaus sind implantierte bzw. implantierbare Herzschrittmacher bekannt, die vollständig in dem Körper eingesetzt sind.

Die vorliegende Erfindung betrifft sowohl externe als auch implantierbare Herzschrittmacher.

Wie ausgeführt werden je nach Anwendungsfall unterschiedliche Typen von Herzschrittmachern eingesetzt. So sind beispielsweise Einkammerschrittmacher (VVI) bekannt, die bei fehlender Herzaktivität im Ventrikel nach Ablauf eines Stimulationsintervalls einen Impuls in dem Ventrikel abgeben. Des Weiteren sind Vorhofschrittmacher (AAI) bekannt, die das Gegenstück zu dem WI-Schrittmacher im Atrium bilden.

Darüber hinaus sind Zweikammerschrittmacher bekannt, beispielsweise VAT-Schrittmacher, die Impulse im Vorhof, d.h. dem Atrium detektieren und dann ggf. eine Stimulation im Ventrikel, d.h. in der Herzkammer abgeben.

Aus dem Stand der Technik sind darüber hinaus sogenannte DDD-Schrittmacher bekannt, die eine Vereinigung der vorgenannten Schrittmachertypen VVI, AAI und VAT darstellen. Diese DDD-Schrittmacher detektieren die Herzaktivität im Atrium und stimulieren dort, wenn keine Eigenaktivität des Herzens wahrgenommen wurde. Darüber hinaus gibt der Schrittmacher einen Impuls im Ventrikel ab, wenn beispielsweise nach einem AV-Intervall nach einer stimulierten Aktion oder einer Herzeigenaktivität im Vorhof in der Herzkammer keine Aktion wahrgenommen wird. Üblicherweise wird im DDD-Modus der rechte Vorhof und die rechte Kammer überwacht und bei Bedarf jeweils stimuliert.

Die DE 20 2010 011244 U1 zeigt einen Kardioverter zur Beseitigung von Vorhofflimmern, der einen Stimulator aufweist, um die Vorhöfe und Ventrikel in der üblichen Weise zu stimulieren.

US 5 324 310 A offenbart einen Herzschrittmacher, der die Merkmale aus dem Oberbegriff des Anspruchs 1 aufweist.
Nach Herzoperationen tritt relativ häufig das sogenannte Vorhofflimmern auf, das als wesentlicher Grund für die postoperative Mortalität gilt.
Der vorliegenden Erfindung liegt die Aufgabe zugrunde, einen Herzschrittmacher der eingangs genannten Art dahingehend weiterzubilden, dass die Auftrittswahrscheinlichkeit von postoperativem Vorhofflimmern und damit die Sterblichkeitsrate verringert wird.
Diese Aufgabe wird durch einen Herzschrittmacher mit den Merkmalen des Anspruchs 1 gelöst. Danach ist vorgesehen, dass die Stimulationseinheit derart ausgebildet ist, dass diese nach einer Stimulation in einem Atrium zeitverzögert zusätzlich eine Stimulation in dem anderen Atrium vornimmt oder veranlasst. Vorgesehen ist, dass nach jedem abgegebenen Impuls, beispielsweise im rechten Atrium, grundsätzlich ein zeitverzögerter weiterer Impuls beispielsweise im linken Atrium abgegeben wird (Triggerung). Ferner ist vorgesehen, dass die Stimulationseinheit derart ausgebildet ist, dass sie eine Stimulation in einem Ventrikel vornimmt oder veranlasst wenn dort durch die Detektionseinheit keine Herzeienaktivität wahrgenommen wird. Alle im Folgenden genannten Beispiele und Ausführungen, die nicht unter den unabhängigen Anspruch 1 fallen, sind nicht Teil der Erfindung. Der aus dem Stand der Technik bekannte und oben näher beschriebene DDD-Modus wird somit in dieser Ausführungsform der Erfindung dahingehend erweitert, dass nach jedem abgegebenen Impuls, beispielsweise im rechten Atrium, grundsätzlich ein zeitverzögerter weiterer Impuls beispielsweise im linken Atrium abgegeben wird (Triggerung).

Der Herzschrittmacher gemäß der vorliegenden Erfindung ist somit vorzugsweise mit Mitteln ausgeführt, die in der Lage sind, diesen erweiterten DDD-Modus auszuführen.

In Anlehnung an den NBG-Code wird dieser Schrittmachermodus auch als DDD-AAT-Modus bezeichnet, wobei die Abkürzung AAT für den Stimulationsort (Atrium), für den Detektionsort (Atrium) und für die Betriebsart (getriggert) steht.

Der Herzschrittmacher gemäß der vorliegenden Erfindung kann als solcher ausgeführt sein oder mit einem Kardioversionsgerät bzw. Defibrillator ausgeführt bzw. verbunden sein.

Die Stimulationseinheit sowie die Detektionseinheit im Sinne der vorliegenden Erfindung ist ein Bestandteil des Herzschrittmachers und kann auch die Elektrode(n) umfassen. Von der Erfindung ist jedoch auch der Fall umfasst, dass die Stimulationseinheit und/oder die Detektionseinheit nicht die Elektrode(n) umfasst, sondern beispielsweise einen Ausgang bzw. Port etc. aufweist, an den die Elektrode(n) angeschlossen werden können.

Vorzugsweise ist vorgesehen, dass die Stimulationseinheit derart ausgebildet ist, dass die Stimulation in dem einen und in dem anderen Atrium unterbleibt, wenn in einem Atrium durch die Detektionseinheit eine Herzeigenaktivität erfasst wurde.

Denkbar ist es somit beispielsweise, dass bei einer Eigenaktivität des Herzens im rechten Vorhof weder eine Stimulation des rechten noch des linken Vorhofs erfolgt.

Vorzugsweise ist vorgesehen, dass eine Wahrnehmungsfunktion von Impulsen nur in einem Atrium vorgenommen wird. Denkbar ist es beispielsweise, auf eine Wahrnehmungsfunktion im linken Vorhof zu verzichten. In diesem Fall verfügt der erfindungsgemäße Herzschrittmacher somit über keine Wahrnehmungsfunktion in einem Vorhof.

In einer weiteren Ausgestaltung der Erfindung ist vorgesehen, dass die Stimulationseinheit derart ausgebildet ist, dass der Zeitabstand zwischen der Stimulation des einen und des anderen Atriums vorzugsweise über ein Menü einstellbar ist. So ist es beispielsweise denkbar, dass über eine geeignete Software der Bediener bzw. das behandelnde Personal die Zeitspanne bzw. die Verzögerung zwischen der Stimulation des rechten und des linken Atriums einstellen kann.

Denkbar ist beispielsweise eine Einstellung in der Spanne > 0ms und 100ms und vorzugsweise in der Spanne > 0ms und 50ms.

Im eingesetzten Zustand ist der Herzschrittmacher derart mit dem Herzen verbunden, dass die Mittel zur Abgabe eines Stimulationsimpulses sowie die Mittel zur Wahrnehmung von Impulsen mit dem rechten Atrium, mit dem linken Atrium und mit einer Herzkammer und vorzugsweise mit der rechten Herzkammer in Verbindung stehen.

Denkbar ist es, dass die Mittel zur Abgabe eines Stimulationsimpulses sowie die Mittel zur Wahrnehmung von Impulsen so angeordnet sind, dass in einer Kammer, beispielsweise in der rechten Kammer und in einem Vorhof, beispielsweise im rechten Vorhof eine Überwachung und eine Stimulation stattfindet, wohingegen im anderen Vorhof, beispielsweise im linken Vorhof keine Wahrnehmungsfunktion vorgesehen ist, sondern dort nur eine Stimulation möglich ist.

Diese Stimulation beispielsweise des linken Vorhofes erfolgt erfindungsgemäß zeitverzögert zu dem abgegebenen Impuls im anderen Atrium, beispielsweise im rechten Atrium.

Die Mittel zur Abgabe wenigstens eines Stimulationsimpulses sowie auch die Detektionsmittel zur Wahrnehmung von Impulsen weisen üblicherweise eine oder mehrere Elektroden auf bzw. stehen mit diesen in Verbindung. Vorzugsweise handelt es sich dabei um bipolare Elektroden, die über eine kathodische Spitze verfügen und eine Anode aufweisen, die üblicherweise in einem bestimmten Abstand von der Spitze entfernt angeordnet ist. Jedoch sind auch unipolare Elektroden von der Erfindung mit umfasst.

Die Mittel zur Wahrnehmung von Impulsen können so ausgeführt sein, dass sie die Herzeigenaktivität und/oder durch den Herzschrittmacher abgegebene Impulse erfasst. Der Begriff "Impulse" im Sinne der vorliegenden Erfindung ist somit nicht auf seitens des Herzschrittmachers abgegebene Impulse beschränkt, sondern umfasst auch die Herzeigenaktivität.

Vorzugsweise ist vorgesehen, dass der Herzschrittmacher über Elektroden verfügt und dass Schaltmittel vorgesehen sind, die derart ausgebildet sind, dass durch die Schaltmittel die indifferenten Pole insbesondere von wenigstens zwei bipolaren Elektroden kurzgeschlossen werden können. Bei diesen indifferenten Polen handelt es sich um die Anoden der Elektroden oder im Falle von unipolaren Elektroden um Gegenelektroden, die beispielsweise durch das Schrittmachergehäuse gebildet werden können.

Eine solche Maßnahme kann beispielsweise durch einen oder mehrere Schalter des Herzschrittmachers erfolgen. So ist es denkbar, dass die indifferenten Pole, d.h. die Anoden aller Kanäle (z.B. rechter und linker Vorhof, rechte Kammer) optional hardwaremäßig kurzgeschlossen werden und somit eine gemeinsame Masse bilden. Vorzugsweise werden bipolare Elektroden eingesetzt. Von der Erfindung ist jedoch auch der Einsatz von unipolaren Elektroden mit umfasst, deren Elektrode, vorzugsweise deren Elektrodenspitze als Kathode dient und bei denen beispielsweise das Schrittmachergehäuse oder eine andere Gegenelektrode die Anode bildet.

Denkbar ist es, dass die Ansteuerung des oder der Schaltmittel über eine Software erfolgt.

Ein solcher Zusammenschluss kann sinnvoll sein, um die klassische Stimulation mit unipolaren Schrittmachern nachzubilden und unkontrollierbaren Spannungsdifferenzen zwischen den Anoden vorzubeugen, die nach der Stimulation zu Fehldetektionen führen könnten. Ein weiterer Vorteil ergibt sich aus der Reduktion der Reizschwellen.

Auch während der Überwachung bzw. in der Wahrnehmung können die indifferenten Pole optional kurzgeschlossen werden, sofern dies erforderlich ist.

Das erfindungsgemäße automatisierte Kurzschließen der indifferenten Pole kann vorzugsweise bei dem erfindungsgemäßen DDD-AAT-Modus angewandt werden, ist jedoch darauf nicht beschränkt und kann auch bei anderen Modi bzw. vorzugsweise Herzschrittmachern, beispielsweise bei Dreikammerschrittmachermodi oder auch bei Zweikammerschrittmachermodi umgesetzt werden.

Die vorliegende Erfindung betrifft des Weiteren ein Verfahren zum Betreiben eines Herzschrittmachers, wobei der Herzschrittmacher eine Stimulation in einem Atrium vornimmt, wenn dort keine Herzeigenaktivität wahrgenommen wurde. Das erfindungsgemäße Verfahren ist so ausgeführt, dass der Herzschrittmacher nach einer Stimulation in einem Atrium zeitverzögert zusätzlich eine Stimulation in dem anderen Atrium vornimmt.

Vorzugsweise ist das Verfahren derart ausgebildet, dass eine Stimulation in einem Ventrikel vorgenommen wird, wenn dort keine Herzeigenaktivität wahrgenommen wurde.

In einem weiteren Verfahren gemäß der Erfindung ist vorgesehen, dass in wenigstens einem Betriebsmodus, vorzugsweise in dem genannten DDD-AAT-Modus wenigstens zwei indifferente Pole, vorzugsweise drei indifferente Pole der Elektroden kurzgeschlossen werden, um die oben genannten Vorteile zu erhalten, die sich mit der dann vorliegenden gemeinsamen Masse ergeben.

Die vorliegende Erfindung betrifft des Weiteren eine auf einem Datenträger gespeicherte Steuerungssoftware zur Durchführung eines erfindungsgemäßen Verfahrens vorzugsweise auf einem Herzschrittmacher gemäß der Erfindung.

Weitere Einzelheiten und Vorteile der Erfindung werden anhand eines im Folgenden beschriebenen Ausführungsbeispiels erläutert.

Das Ausführungsbeispiel betrifft einen externen Herzschrittmacher, der über drei bipolare Elektroden mit dem Herz eines Patienten verbindbar bzw. verbunden ist.

Der Herzschrittmacher gemäß dem Ausführungsbeispiel verfügt über eine Stimulationseinheit, die Mittel zur Abgabe wenigstens eines Stimulationsimpulses an die drei Elektroden aufweist oder diese Elektroden umfasst, und über wenigstens eine Detektionseinheit, die Mittel zur Wahrnehmung von Impulsen von zwei der genannten Elektroden aufweist oder diese Elektroden umfasst.

Alle oder ein Teil der Elektroden dienen somit nicht nur zur Abgabe von Stimulationsimpulsen sondern auch zur Detektion der Herzeigenaktivität.

Der Schrittmacher umfasst darüber hinaus eine Kontroll- und Steuereinheit, die einerseits Signale von der Detektionseinheit empfängt und andererseits Signale an die Stimulationseinheit abgibt.

Erfindungsgemäß ist vorgesehen, dass diese Kontroll- und Steuereinheit so ausgeführt ist, dass der Herzschrittmacher in dem DDD-AAT-Modus betreibbar ist. In diesem Modus wird durch eine erste Elektrode die Herzeigenaktivität in einem Atrium, vorzugsweise im rechten Atrium überwacht und bei Ausbleiben der dortigen Herzeigenaktivität wird ein Impuls an das Atrium abgegeben.

Des Weiteren wird überprüft, ob beispielsweise nach einem eingestellten AV-Intervall nach der stimulierten oder detektierten Vorhofaktion im Ventrikel, beispielsweise in der rechten Kammer eine Herzeigenaktivität wahrgenommen werden kann. Ist dies nicht der Fall, gibt der Herzschrittmacher dort über die Stimulationseinheit ebenfalls einen Impuls ab.

Eine Herzeigenaktivität in dem genannten Atrium oder Ventrikel führt zur Unterdrückung der Impulsabgabe in der jeweiligen Kammer (Inhibition). Für die Wahrnehmungsfunktion und die Stimulation des Ventrikels ist eine zweite Elektrode vorgesehen.

Des Weiteren ist eine dritte Elektrode vorgesehen, die mit dem anderen Atrium, d.h. beispielsweise mit dem linken Atrium derart verbunden ist, dass sie dort Impulse abgeben kann. Diese Impulsabgabe erfolgt vorzugsweise nach jedem abgegebenen Impuls im anderen, vorzugsweise im rechten Atrium und zwar zeitverzögert.

Der Vorhof, in dem dieser zeitverzögerte Impuls abgegeben wird, wird vorzugsweise nicht überwacht, d.h. dort findet in bevorzugter Ausgestaltung keine Wahrnehmungsfunktion, d.h. keine Detektion der Herzeigenaktivität statt. Dies erfolgt nur in dem anderen Vorhof, beispielsweise im rechten Vorhof. Wird dort eine Herzeigenaktivität festgestellt, erfolgt weder eine Stimulation des rechten noch des linken Vorhofs.

In einer weiteren Ausgestaltung der Erfindung ist es denkbar, dass in wenigstens einem Betriebszustand und wenigstens zeitweise eine hardwaremäßige Zusammenschaltung der indifferenten Pole, d.h. der Anoden aller dreier Kanäle erfolgt. Diese bilden dann die gemeinsame Masse. Wie bereits oben ausgeführt kann dieser Zusammenschluss sinnvoll sein um die klassische Stimulation mit unipolaren Schrittmachern nachzubilden und um unkontrollierbaren Spannungsdifferenzen zwischen den Anoden vorzubeugen.

Dementsprechend weist der Herzschrittmacher in diesem Ausführungsbeispiel Schaltmittel auf, durch die die indifferenten Pole im Falle von unipolaren oder bipolaren Elektroden zusammenschaltbar sind. Dies kann in Zusammenhang mit dem beschriebenen DDD-AAT-Modus oder auch bei anderen Modi bzw. bei anderen Herzschrittmachern zum Einsatz kommen.
Wie oben ausgeführt, kann es sich bei dem Herzschrittmacher gemäß der Erfindung um einen externen oder auch um einen implantierbaren Herzschrittmacher handeln. Entsprechendes gilt für das Verfahren.

## Patentansprüche

1. Herzschrittmacher zur Stimulation eines Herzens, umfassend:
eine Stimulationseinheit mit Mitteln zur Abgabe eines Stimulationsimpulses, und
eine Detektionseinheit mit Mitteln zur Wahrnehmung eines Impulses, wobei
wenn die Stimulationseinheit mit dem Herzen verbunden ist, diese dazu ausgelegt ist, einen Stimulationsimpuls an ein Atrium abzugeben, wenn dort durch die Detektionseinheit keine Herzeigenaktivität wahrgenommen wurde,
**dadurch gekennzeichnet, dass**
die Stimulationseinheit dazu ausgelegt ist, nach jedem Abgeben eines Stimulationsimpulses in dem Atrium zeitverzögert zusätzlich einen Stimulationsimpuls an das andere Atrium des Herzens abzugeben, wobei die Stimulationseinheit derart ausgebildet ist, einen Stimulationsimpuls in einem Ventrikel abzugeben, wenn dort durch die Detektionseinheit keine Herzeigenaktivität wahrgenommen wurde.

2. Herzschrittmacher nach Anspruch 1, **dadurch gekennzeichnet, dass** die Stimulationseinheit derart ausgebildet ist, dass die Stimulation sowohl in dem einen als auch in dem anderen Atrium unterbleibt, wenn in einem Atrium durch die Detektionseinheit eine Herzeigenaktivität erfasst wurde.

3. Herzschrittmacher nach einem der vorhergehenden Ansprüche, **dadurch gekennzeichnet, dass** die Detektionseinheit derart ausgebildet ist, dass sie die Wahrnehmung von Impulsen nur in einem Atrium vornimmt und/oder dass die Stimulationseinheit derart ausgebildet ist, dass der Zeitabstand zwischen der Stimulation des einen und des anderen Atriums vorzugsweise über ein Menü einstellbar ist.

4. Herzschrittmacher nach einem der vorhergehenden Ansprüche, **dadurch gekennzeichnet, dass** die Stimulationseinheit derart ausgebildet ist, dass der Zeitabstand zwischen der Stimulation des einen und des anderen Atriums in der Spanne zwischen > 0 ms und 100 ms, vorzugsweise in der Spanne zwischen > 0 ms und 50 ms liegt.

5. Herzschrittmacher nach einem der vorhergehenden Ansprüche, **dadurch gekennzeichnet, dass** die Mittel zur Abgabe eines Stimulationsimpulses sowie die Mittel zur Wahrnehmung von Impulsen mit dem rechten Atrium, mit dem linken Atrium und mit einer Herzkammer, vorzugsweise mit der rechten Herzkammer in Verbindung stehen.

6. Herzschrittmacher nach einem der vorhergehenden Ansprüche, **dadurch gekennzeichnet, dass** die Mittel zur Abgabe wenigstens eines Stimulationsimpulses und/oder die Mittel zur Wahrnehmung eines Impulses eine oder mehrere Elektroden umfassen, und dass Schaltmittel vorgesehenen sind, die derart ausgebildet sind, dass mittels der Schaltmittel in wenigstens einem Zustand der Schaltmittel die indifferenten Pole von wenigstens zwei Elektroden kurzgeschlossen sind.

7. Herzschrittmacher nach Anspruch 6, **dadurch gekennzeichnet, dass** die Schaltmittel derart ausgebildet sind, dass die indifferenten Pole dreier Elektroden kurzgeschlossen sind und/oder **dadurch gekennzeichnet, dass** die Schaltmittel derart ausgebildet sind, dass sie über eine Software ansteuerbar sind.

## Claims

1. Pacemaker to stimulate a heart, including:
a stimulation unit with means for delivering a stimulation impulse, and
a detection unit with means for perceiving an impulse, wherein
when the stimulation unit is connected with the heart, said unit is adapted to deliver a stimulation impulse to an atrium, if no intrinsic cardiac activity was perceived there by means of the detection unit,
**characterized in that**
the stimulation unit is adapted, after every delivery of a stimulation impulse in the atrium, to additionally deliver a stimulation impulse to the other atrium of the heart in a time-delayed manner, wherein the stimulation unit is formed in such a manner as to deliver a stimulation impulse in a ventricle, if no intrinsic cardiac activity was perceived there by the detection unit.

2. Pacemaker according to claim 1, **characterized in that** the stimulation unit is formed in such a manner that the stimulation is suppressed in the one as well as also in the other atrium, if an intrinsic cardiac activity is detected by the detection unit in one atrium.

3. Pacemaker according to one of the preceding claims, **characterized in that** the detection unit is formed is such a manner that it only performs the perception of impulses in one atrium, and/or **in that** the stimulation unit is formed in such a manner that the time interval between the stimulation of the one and the other atrium can preferably be set via a menu.

4. Pacemaker according to one of the preceding claims, **characterized in that** the stimulation unit is formed in such a manner that the time interval between the stimulation of the one and the other atrium lies in the range between > 0 ms and 100 ms, preferably in the range between > 0 ms and 50 ms.

5. Pacemaker according to one of the preceding claims, **characterized in that** the means for delivering a stimulation impulse, as well as the means to perceive impulses are in connection with the right atrium, with the left atrium, and with a heart chamber, preferably with the right heart chamber.

6. Pacemaker according to one of the preceding claims, **characterized in that** the means for delivering at least one stimulation impulse and/or the means to perceive an impulse include one or multiple electrodes, and **in that** switching means are provided, which are formed in such a manner that, by means of the switching means, the indifferent poles of at least two electrodes are short-circuited, in at least one state of the switching means.

7. Pacemaker according to claim 6, **characterized in that** the switching means are formed in such a manner that the indifferent poles of three electrodes are short-circuited, and/or **characterized in that** the switching means are formed in such a manner that they are controllable via a software.

## Revendications

1. Stimulateur cardiaque destiné à la stimulation d'un coeur, comprenant :
une unité de stimulation comprenant des moyens destinés à émettre une impulsion de stimulation,
et
une unité de détection comprenant des moyens destinés à déceler une impulsion,
l'unité de stimulation, lorsque celle-ci est reliée au coeur, étant conçue pour émettre une impulsion de stimulation à un atrium lorsque l'unité de détection n'y a décelé aucune propre activité du coeur,
**caractérisé en ce que**
l'unité de stimulation est conçue, après chaque émission d'une impulsion de stimulation dans l'atrium, pour émettre de manière temporisée en plus une impulsion de stimulation à l'autre atrium du coeur, l'unité de stimulation étant réalisée de manière à émettre une impulsion de stimulation dans un ventricule lorsqu'aucune propre activité du coeur n'y a été perçue par l'unité de détection.

2. Stimulateur cardiaque selon la revendication 1, **caractérisé en ce que** l'unité de stimulation est réalisée de manière à ce que la stimulation n'ait lieu ni dans l'un ni dans l'autre atrium lorsqu'une propre activité du coeur a été détectée dans un atrium par l'unité de détection.

3. Stimulateur cardiaque selon l'une quelconque des revendications précédentes, **caractérisé en ce que** l'unité de détection est réalisée de manière à déceler les impulsions seulement dans un atrium et/ou **en ce que** l'unité de stimulation est réalisée de manière à ce que l'écart de temps entre la stimulation de l'un et de l'autre atrium soit réglable de préférence par l'intermédiaire d'un menu.

4. Stimulateur cardiaque selon l'une quelconque des revendications précédentes, **caractérisé en ce que** l'unité de stimulation est réalisée de manière à ce que l'écart de temps entre la stimulation de l'un et de l'autre atrium soit situé dans la marge comprise entre > 0 ms et 100 ms, de préférence dans la marge comprise entre > 0 ms et 50 ms.

5. Stimulateur cardiaque selon l'une quelconque des revendications précédentes, **caractérisé en ce que** les moyens d'émission d'une impulsion de stimulation ainsi que les moyens de détection d'impulsions sont en liaison avec l'atrium droit, avec l'atrium gauche et avec un ventricule, de préférence avec le ventricule droit.

6. Stimulateur cardiaque selon l'une quelconque des revendications précédentes, **caractérisé en ce que** les moyens d'émission d'au moins une impulsion de stimulation ainsi que les moyens de détection d'une impulsion comprennent une ou plusieurs électrodes, et **en ce que** des moyens de commutation sont ménagés, lesquels sont réalisés de manière à ce qu'à l'aide des moyens de commutation, les pôles indifférents d'au moins deux électrodes soient court-circuités dans au moins un état des moyens de commutation.

7. Stimulateur cardiaque selon la revendication 6, **caractérisé en ce que** les moyens de commutation sont réalisés de manière à ce que les pôles indifférents de trois électrodes soient court-circuités et/ou **caractérisé en ce que** les moyens de commutation sont réalisés de manière à pouvoir être commandés par l'intermédiaire d'un logiciel.
